# EUROPEAN PATENT APPLICATION

(11) **EP 0 582 541 A2**
(43) Date of publication of application: **09.02.1994**
(21) Application number: 93810302.5
(22) Date of filing: 27.04.1993
(51) Int. Cl.: C12N 15/00, C12N 15/32, C12N 1/21, A01N 63/00

(54) **Process for creating conjugation-proficient Bacillus thuringiensis strains**

(30) Priority: 01.05.1992 US 877008
(71) Applicant: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Piot, Jean-Christophe, Menlo Park, CA 94025 (US)

(57) **Abstract**

The invention concerns a process for creating conjugation-proficient Bacillus thuringiensis strains useful in plasmid transfer between strains of Bacillus thuringiensis.

## Description

The present invention relates to new strains of bacteria producing insecticidal toxins, processes for their preparation and their use in combatting insect pests.

More particularly, the invention relates to Bacillus thuringiensis (hereinafter B.t.) hybrids having improved insecticidal properties.

Biological insecticides in general and B.t. in particular have in recent years found increasing use as specialized alternatives to broad spectrum chemical pesticides due to their occurrence in nature and their high host specificity.

The increased acceptance of such products has at the same time led to more sophisticated demands with regard to spectrum of activity against target pests and ability to spare non-target insects.

Various methods of achieving this result are known such as
a) isolation of strains containing mutated toxin-encoding genes where the mutation may be spontaneous or induced (cf GB Patent Application 2216127A);
b) Transformation of strains using techniques such as electroporation or electrotransformation to introduce genes - either natural, mutated or constructed - which express a desired endotoxin (cf Bone & Ellar, FEMS Microbiology letters 58 p. 171-178 (1989)).
c) Conjugation of naturally occurring strains or mutated strains to produce hybrid strains containing plasmids from both parental strains having differing or complementary host specificities (cf USP 4,797,279).

In this latter case however problems are often encountered in that the plasmid(s) bearing the sought-after gene(s) is (are) present in a strain which is not conjugation-proficient. (Conjugation-proficient strains being those which possess the ability to act efficiently as donors of at least one of the plasmids they contain.) This usually has as a result that either the desired plasmids are not transferred when attempting conjugation or that the roles of the donor and recipient cell are reversed leading to a hybrid which lacks the genome of the originally desired recipient and is thus deficient in other ways e.g. does not grow well, expresses toxin poorly, is difficult to formulate, or the like.

An example of a desirable conjugation which is beset by this problem is the inability until now to create a B.t. kurstaki strain containing a cry IC gene.

Reddy et al describe interspecies transfer of plasmids between B.t., Bacillus cereus and Bacillus anthracis but do not disclose intraspecies transfer. It has now been found according to the invention that a conjugation deficient (c-d) B.t. strain containing a desired plasmid can be converted into a conjugation proficient (c-p) strain by conjugating it with a second B.t. strain which is conjugation proficient. The transconjugant strain thus obtained, which will be both c-p and will contain the desired plasmid bearing the sought-after gene, may then be used as a donor cell to conjugate with a desired host.

By a conjugation-deficient B.t. strain is meant one exhibiting plasmid transfer at a frequency of less than about 2%, and more preferably one exhibiting a plasmid transfer frequency of less than about 1%.

According to the present invention there is provided a process for converting a conjugation-deficient B.t. strain into a conjugation-proficient strain comprising conjugating the conjugation-deficient B.t. strain with a conjugation-proficient B.t. strain as donor.

In another aspect the invention concerns a process for transferring a plasmid from a conjugation deficient B.t. strain to a desired host strain which comprises a) conjugating the conjugation deficient B.t. strain with a conjugation-proficient B.t. strain as donor; b) using the B.t. strain thus obtained as donor strain for conjugation with a desired host strain.

In the event that it is desirable to further modify or add to the plasmid content of the B.t. strain obtained following conjugation according to b) above it may in turn be conjugated with a further B.t. strain having some or all of the plasmids and/or other genomic features of the desired final B.t. strain, either as donor or as recipient.

Conjugation is effected in conventional manner such as described in Gonzalez J.M. and Carlton B.C., 1982, Plasmid Transfer in *Bacillus thuringiensis,* 85-95, in Genetic and Cellular Technology, edited by L.P. Gage, Volume 1 Genetic Exchange, edited by U.N. Streips et.al. Marcel Dekker, Inc. New York and Basel; Fischer H.-M. 1983, Plasmid und Plasmidubertragung bei *Bacillus thuringiensis,* PHD Thesis ETH Nr. 7375, Swiss Federal Institute of Technology, ADAG Administration & Druck AG, 83 pages; USP 4,797,279 and EP 178151, the contents of which in this respect are incorporated by reference.

The choice of c-p and (c-d) strains and subsequent recipient or donor strains will of course depend upon the desired properties of the final products.

With respect to spectrum and level of activity strains are chosen containing plasmids bearing genes which encode for insecticidal endotoxins having the desired specificity and level of activity. For a discussion of suitable genes and B.t. strains harboring them see Hofte and Whiteley, Microbial Reviews, v. 53 N. 2 pp. 242-255 (June 1989) the contents of which in this respect are incorporated herein by reference.

Examples of genes are those encoding endotoxins with activity against eg Lepidoptera (eg Spdoptera spp.), Diptera and/or Coleoptera. The plasmids may also contain genes encoding the useful proteins such as those capable of enhancing the activity of the endotoxins.

The process according to the invention can be used for example to achieve conjugative transfer of a cry IC gene into any species of B.t. not containing that gene. Particularly employing the process according to the invention has for the first time provided for the preparation of a B.t. kurstaki strain harboring a cry IC gene.

The invention therefore further concerns a B.t. kurstaki strain comprising a cry IC gene.

As used herein the term B.t. kurstaki strains refers to B.t. strains which are characterized by a flagellar serotype 3a3b.

In the following non-limiting conjugation example temperatures are given in centigrade. Other combinations of genes and strains producing the desired activity according to the invention may be constructed and employed analogously or as otherwise described herein.

### EXAMPLE 1

### a) Construction of a B.t. entomocidus6.1 donor strain

1 ml of a culture of a B.t. kurstaki HD-73 strain (available from the Agricultural Research Culture Collection (NRRL), Peoria, IL 51604 under the accession number NRRL B-4488) containing the conjugative plasmid pAMβ1 (containing an erythromycin resistance gene) of Streptococcus faecalis and 1 ml of a culture of a spontaneous streptomycin resistant mutant of B.t. entomocidus 6.1 (available from the National Collection of Microorganisms Cultures (CNCM), Paris under the accession number 1-660) - each in exponential growth phase (about 5x10⁷ cells/ml) - are mixed, and filtered on a 0.45 µm cellulose acetate membrane filter. The filter is laid on a Luria Medium plate (LA: 15 g Bacto Agar, Difco; 10 g Bacto Tryptone, Difco; 5 g Bacto Yeast Extract, Difco; 10 g NaCl; in 1 L. distilled water) and incubated for 6 to 24 hours at 30°C.

The filter is transferred to 1 ml Luria Broth (LB: same as above, without agar) and the cells are resuspended by vigorous shaking. Aliquots are plated onto LA plates containing 1 mg/ml streptomycin and 50 µg/ml erythromycin to select the transconjugants.

The thus obtained transconjugants are reisolated, and occurrence of the 50 MDa transmissible plasmid, originating from B.t. kurstaki HD-73, in the transconjugant clones is determined by plasmid pattern analysis, or by amplification, by Polymerase Chain Reaction (PCR), of a sequence of DNA belonging to the cry IA(c) gene carried by this plasmid. The presence of the cry IC gene in the cry IA(c)-containing transconjugants is ascertained by PCR amplification of a portion of the cry IC gene, and transconjugant strains containing both genes are selected as B.t. entomocidus 6.1 donor strains. It must be noted that some other plasmids from B.t. kurstaki HD-73, such as the 5.2 and the 5.6 MDa plasmids, can also be transferred in the process. The strain cg89.7.44 obtained in this example and used in the subsequent conjugation also contains those two plasmids.

### b) Construction of a B.t. kurstaki recipient strain

Plasmid pBC16.1 which carries a tetracycline resistance gene (Kreft et.al. Mol. Gen. Genet. 162, 59-67) is introduced into B.t. kurstaki strain [HD562] obtainable from the USDA using an electroporation method such as described in Bone & Ellar (FEMS Microbiology Letters 58, p 171-178 (1989) the contents of which in this respect are incorporated herein by reference.Selection of transformed cells takes place on LA plates containing 50 µg/ml tetracycline. One such strain is designated HD562 (pBC16.1). Other methods allowing the construction of a recipient strain such as selection of spontaneous or induced antibiotic resistant mutants may also be used.

### c) Conjugative transfer of cry IC in B.t. kurstaki HD562

Conjugation of strains cg89.7.44 and HD562 (pBC16.1) is performed as described in the first part a) above. The transconjugant strains are isolated by plating aliquots of the cell suspension recovered from the filter on LA plates containing 50 µg/ml erythromycin and 25 µg/ml tetracycline. Clones able to grow under these conditions contain both pBC16.1 and pAMβ1 plasmids. To overrule the possibility that pBC16.1 has been transferred from HD562 to cg89.7.44, the transconjugant clones are transferred to LA plates containing streptomycin. Only derivatives of cg89.7.44 can grow on this media, and this screening allows elimination of these false positives.

The thus selected erythromycin and tetracycline resistant HD562 derivatives are isolated and their content of cry genes is analyzed by PCR amplification, using sets of primers specific for each type of cry gene. HD562 derivatives containing the cry IC gene are obtained by these means, all of which have lost the cry IA(c) and cry II A genes probably because of plasmid incompatibility. The clone cg9065.274 is one of these derivatives.

### d) Reintroduction of cryIA(c) in cryIC-containing transconjugants

Since cry IA(c) is the gene whose product is the most active against a wide range of lepidopteran pests, it has been reintroduced in the cry IC-containing transconjugants obtained eg analogously to c) above in a third conjugation. First, derivatives having spontaneously lost the pAMβ1 plasmid, which is used as a marker for conjugative transfer, are isolated by restreaking randomly chosen clones, growing on a tetracycline-containing LA plate, onto a tetracyclin and erythromycin-containing LA plate. Clones that have lost the pAMβ1 plasmid cannot grow on this media and are isolated accordingly. The clone cg9065.274.1 is an example of such a clone.

The next step involves reintroduction of the cry IA(c) gene into strain 9065.274.1. This can be done by conjugating the donor strain cg89.7.44 with the recipient strain cg9065.274.1 in order to transfer the 50 MDa plasmid from B.t. kurstaki HD-73 containing the cry IA(c) gene. Transconjugant clones are again selected on LA plates containing both erythromycin and tetracycline, and their belonging to HD562 is demonstrated by their sensitivity to streptomycin, as described above. The cry gene content of the transconjugant clones is analyzed by PCR amplification, using sets of primers specific for each type of cry genes. HD562 derivatives containing both cry IA(c) and cry IC are isolated in this fashion, most of which have lost the cry IA(b) gene, probably because of some incompatibility of the plasmids carrying cry IA(c) and cry IA(b).

It is thus more desirable to reintroduce the cry IA(c) gene on a plasmid known to be compatible with all the plasmids carrying cry genes, more precisely with the plasmids carrying the cry IA(b) gene. Since the cry IA(c)-carrying 50MDa plasmid and the cry IA(b)-carrying plasmid of strain HD562 are incompatible, it is possible to screen B.t. isolates for the presence of a cry IA(c) gene and the absence of DNA sequences contained in the origin of replication of the 50MDa plasmid from HD73. The sequences are the subject of co-pending US Patent Application 07/736,668 filed July 26, 1991 the contents of which in this respect are incorporated by reference. Oligonucleotide primers can be designed and synthesized to enable the detection of these sequences, and B.t. strains can be selected on the basis of the presence of the cry IA(c) gene and the absence of sequences homologous to the origin of replication of the 50 MDa plasmid of HD73. B.t. kurstaki strain HD89 is such a strain.

### Construction of an HD89 donor strain

This can be carried out analogously to Example 1a) above replacing B.t. entomocidus 6.1 by B.t. kurstaki HD89. The occurrence of the 50 MDa transmissible plasmid originating from B.t. kurstaki HD73 in the transconjugant clones is determined by amplification by PCR of a sequence of DNA homologous to the origin of replication of the 50 MDa plasmid of HD73. The presence of the 50 MDa plasmid can alternatively be ascertained by agarose gel electrophoresis of plasmids DNA extracts. The integrity of the original plasmid complement of strain HD89 can also be ascertained in the same manner. Strain cg 91043.67 is such a strain.

### Construction of the final strain by Transfert of the cry IA(c)-carrying plasmid from HD89 into cg9065.274

Transfer of the cry IA(c)-containing plasmid of HD89 is achieved by conjugating strain cg91043.67 with the recipient strain cg9065.274.1. This is carried out analogously to Example 1(c) above. HD562 derivatives containing cry IA(a), cry IA(b), cry IA(c), cry IC and cry IIA are isolated in this fashion. Strain cg92004.24 is such a strain.

### BIOLOGICAL ACTIVITY

Usefulness of the transconjugant HD562 derivatives is shown by their improved insecticidal activity on Spodoptera spp, such as Spodoptera exigua, while retaining their original level of activity against the more susceptible lepidopteran pests such as Trichoplusia ni and Heliothis zea.

| Strain | LC50 on Spod. exigua |
|---|---|
| cg 92004.24 | 1140 |
| HD562 | 1890 |

LC50 is expressed in ppm of sporulated culture in the insect diet.

The transconjugates are especialy useful in resistance management for the control of insect populations which have acquired resistance to conventional B.t. based pesticides.

The invention therefore also provides a method of combatting insects comprising applying to the insects or their habitat an insecticidally effective amount of a B.t. hybrid whenever prepared by a process according to the invention. The invention further provides a method of combatting insects comprising applying to the insects or their habitat an insecticidally effective amount of a B.t. kurstaki strain comprising a cry IC gene. Such B.t. hybrids are conveniently employed in insecticidal composition form, e.g. in suspension concentrate form or powder form. Such compositions contain suitable diluents and preferably additional insecticidally acceptable additives such as UV protecting agents, surfactants, emulsifiers, etc. They are prepared in conventional manner according to known B.t. strains containing biological insecticides.

The term diluents as used herein means liquid or solid, agriculturally acceptable material, which may be added to the B.t. hybrids of the invention to bring them in an easier or better applicable form, respectively to dilute the active agent to a usable or desirable strength of activity.

It will be appreciated that such B.t. hybrids once obtained by conjugation will be more economically reproduced/multiplied by fermentation in an appropriate nutritive medium comprising a nitrogen source (e.g. fish meal), a carbohydrate source (e.g. starch) and mineral salts and adjusted to a pH of about 7.

Such fermentation is conveniently effected at a temperature of 20° to 40°C, e.g. 30°C. A suitable fermentation time is from 24 to 72 hours, e.g. 36 hours.

A suitable suspension concentrate of the B.t. hybrids of the invention can be obtained by evaporation of the fermentation liquor to the desired concentration. Additives may be added where desired.

Analogously, a wettable powder formulation may be obtained by spray drying the fermentation liquor, comprising optionally emulsifiers, UV protectants and the like, and pulverization of the thus obtained solid.

The thus obtained formulations contain a substantial part of the components of the nutritive medium as diluent.

Alternatively, the fermentation liquor may be centrifugated to separate the bigger particles of the nutritive medium and then be converted, as indicated above, to a suitable suspension concentrate or wettable powder formulation. Suitable formulation comprise e.g. between 1,000 to 40,000 IU per mg or ml of formulated product.

### SOLUBLE CONCENTRATE

An aqueous nutrient medium containing about 1.86% beet molasses, 1.4% oil-free cottonseed endosperm flour, 1.7% corn steep liquor solids and 0.1% calcium carbonate is formed, the pH adjusted to within the range of 7.2 to 7.6 the batch sterilized for 20 to 30 minutes at 121°C and then inoculated with 5% of its volume of a B.t. hybrid. The culturing process is run with agitation with about 5 psig back pressure and at an incubation temperature of 30°C. The broth is chilled to 18° to 20°C and its pH adjusted to about 5.5 with concentrated H₂SO₄. The broth is screened through a screen having 150 meshes to the square inch, the resulting screened culture centrifuged to remove part of the impurities and evaporated to a concentrate volume having the desired potency. To this concentrate are added 0.4% by weight of an emulsifier (e.g. isoctyl phenyl polyoxyethanol) and 0.8% by weight of a UV absorber.

### WETTABLE POWDER

The evaporated concentrate volume obtained according to the preceding example is spray dried, and the thus obtained technical concentrated blended with a "carrier mixture" consisting of 10% silica and 90% soybean protein, the amount of carrier mixture depending on the potency of the technical concentrate and the desired potency of the wettable powder.

To this wettable powder may be added 0.4% by weight of an emulsifier and 0.8% by weight of a UV absorber.

## Claims

1. A process for converting a conjugation-deficient B.t. strain into a conjugation-proficient strain comprising conjugating the conjugation-deficient B.t. strain with a conjugation-proficient B.t. strain as donor.

2. A process for transferring a plasmid from a conjugation deficient B.t. strain to a desired host strain which comprises a) conjugating the conjugation deficient B.t. strain with a conjugation-proficient B.t. strain as donor; b) using the B.t strain thus obtained as a donor strain for conjugation with a desired strain, such as a B.t. strain with high insecticidal activity or favorable fermentation characteristics.

3. A process according to claim 2, wherein the plasmid contains a gene encoding an insecticidal endotoxin.

4. A process according to either of claims 2 or 3, wherein the plasmid contains a gene encoding an endotoxin having insecticidal activity against Spodoptera spp.

5. A process according to Claim 2, wherein the plasmid contains the cry IC gene or a gene homologous thereto or having similar insecticidal activity and spectrum.

6. A process according to Claim 2, wherein the plasmid contains a gene encoding a substance which enhances the activity of insecticidal crystal proteins.

7. A process according to Claim 2, wherein the plasmid contains a cry IC gene.

8. A B.t. kurstaki strain comprising a cry IC gene.

9. A B.t. strain whenever prepared by a process according to any one of Claims 1, 2 or 7.

10. A method of combatting insects comprising applying to the insects or their habitat an insecticidally effective amount of a B.t. strain according to Claim 9.

11. A method of combatting insects comprising applying to the insects or their habitat an insecticidally effective amount of a B.t. strain according to Claim 8.

12. An insecticidal composition comprising a B.t. strain according to either of Claims 8 or 9, together with a suitable diluent.
